(19) Europäisches Patentamt
European Patent Office
Office européen des brevets

(11) Numéro de publication: **0 522 899 B1**

(12) **FASCICULE DE BREVET EUROPEEN**

(45) Date de publication de fascicule du brevet: **07.06.95**

(51) Int. Cl.[6]: **A61K 7/48**, A61K 7/06, C08J 3/03

(21) Numéro de dépôt: **92401630.6**

(22) Date de dépôt: **12.06.92**

(54) **Compositions cosmétiques se présentant sous forme d'émulsions aqueuses d'organopolysiloxanes.**

(30) Priorité: **09.07.91 FR 9109006**

(43) Date de publication de la demande:
**13.01.93 Bulletin 93/02**

(45) Mention de la délivrance du brevet:
**07.06.95 Bulletin 95/23**

(84) Etats contractants désignés:
**BE DE ES FR GB IT**

(56) Documents cités:
**EP-A- 0 200 916**
**EP-A- 0 268 950**
**EP-A- 0 370 764**
**EP-A- 0 398 177**

(73) Titulaire: **RHONE-POULENC CHIMIE**
**25, Ouai Paul Doumer**
**F-92408 Courbevoie Cédex (FR)**

(72) Inventeur: **Beucherie, Jeannine**
**4, Rue Pasteur**
**F-91000 Massy (FR)**
Inventeur: **Mercier, Jean-Michel**
**2, Résidence Plein-Sud**
**F-94320 Thiais (FR)**

(74) Mandataire: **Fabre, Madeleine-France et al**
**RHONE-POULENC CHIMIE,**
**Direction de la Propriété Industrielle,**
**25 Ouai Paul Doumer**
**F-92408 Courbevoie Cédex (FR)**

**EP 0 522 899 B1**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention est relative à de nouvelles compositions cosmétiques destinées notamment aus soins des cheveux ou de la peau.

Les silicones sont depuis longtemps reconnus comme étant des matières premières aux qualités multiples (toucher, étalement, inertie, etc.....) utilisables avantageusement dans les compositions cosmétiques.

L'application d'émulsions aqueuses à base de silicone conduit à la formation d'un film hydrophobe invisible et cette propriété est particulièrement intéressante en application cutanée (obtention d'un toucher non collant, non gras et doux) et en application capillaire (effet démèlant). Dans ce contexte, on utilise couramment, en raison de leur facilité de mise en émulsion, des huiles silicones de faibles poids moléculaires ayant une viscosité dynamique à 25 °C bien inférieure à $5.10^4$ mPa.s. Il est cependant apparu comme opportun d'utiliser, pour leur propriété de rémanence supérieure, des composés silicones de poids moléculaires et de viscosités plus élevés, comme des huiles et des gommes ayant une viscosité à 25 °C au moins égale à $9.10^4$ mPa.s

Dans la suite du présent mémoire, on désignera par l'expression "composés THV" (ce qui signifie : composés de Très Haute Viscosité), l'ensemble formé par les huiles et les gommes capables de présenter à 25 °C une viscosité aussi élevée que celle au moins égale à $9.10^4$ mPa.s

Au départ de composés THV silicones, la préparation d'émulsions aqueuses à la fois fines et stables présente toutefois certaines difficultés qui peuvent être surmontées, comme cela ressort de l'enseignement de EP-A-0 200 916, à condition d'une part d'engager le composé THV sous forme de solution dans un silicone volatil, ladite solution ayant une viscosité pouvant se situer sans inconvénient dans l'intervalle allant de 30 000 à $2.10^6$ mPa.s ou d'avantage et d'autre part de choisir un mélange judicieux de trois agents tensio-actifs de nature non ionique ayant des valeurs HLB de 7 à 9 pour le premier de ces agents, de 13 à 15 pour le second et égales ou supérieures à 16 pour le troisième de ces agents.

La présente invention a pour but de fournir des compositions cosmétiques :

- qui se présentent sous forme d'émulsions aqueuses fines et stables comprenant un composé THV silicone ayant une viscosité au moins égale à $9.10^4$ mPa.s, et
- qui peuvent avantageusement faire appel pour leur préparation à un seul agent tensio-actif au lieu de trois agents comme il est proposé dans l'art antérieur précité. Par l'expression "émulsions aqueuses fines", on désigne des émulsions dans lesquelles 50 % en volume des globules dispersés ont des dimensions qui sont inférieures à un chiffre aussi petit que celui se situant dans l'intervalle allant de 1 à 4 $\mu$m.

Plus précisément, la présente invention concerne des compositions cosmétiques se présentant sous forme d'émulsions aqueuses silicone/eau, caractérisées par les points suivants :

- point 1 : elles sont préparées en milieu aqueux à partir de constituants comprenant les ingrédients obligatoires suivants :
  (a) : un composé THV polydiorganosiloxane dont la viscosité à 25 °C est au moins égale à $9.10^4$ mPa.s,
  (b) : au moins un composé silicone fluide dont la viscosité à 25 °C est au plus égale à 50 000 mPa.s,
  (c) : un agent tensio-actif non ionique consistant en un sucroglycéride ;
- point 2 : elles comportent :
  - 100 parties en poids de matière silicone constituée par le mélange (a) + (b), le pourcentage pondéral de composé THV (a) dans la matière silicone (a) + (b) représentant 5 à 50 %,
  - 2 à 700 parties en poids d'agent tensio-actif (c), et
  - 3 à 2 000 parties en poids d'eau, avec la condition supplémentaire selon laquelle le rapport parties en poids d'agent tensio-actif (c)/parties en poids d'eau est au plus égal à 0,7.

Les compositions cosmétiques selon la présente invention font appel, pour leur préparation, à un constituant (a) consistant dans un composé THV polydiorganosiloxane comprenant de 0 à 4 % en poids de groupements vinyle et dont la viscosité à 25 °C est au moins égale à $9.10^4$ mPa.s. Ces composés THV sont des polymères linéaires, de poids moléculaire élevé (supérieur à 90 000 g/mole), dont la chaîne polydiorganosiloxane est constituée essentiellement de motifs de formule $(R)_2SiO$. Cette chaîne est bloquée à chaque extrémité par un motif de formule $(R)_3SiO_{0,5}$ et/ou un radical de formule OR'. Dans ces formules :

- les symboles R, identiques ou différents, représentent : des radicaux hydrocarbonés monovalents tels que des radicaux alkyle, par exemple, méthyle, éthyle, propyle, octyle, octadécyle ; des radicaux aryle, par exemple phényle, totyle, xylyle ; des radicaux aralkyle tels que par exemple benzyle, phényléthyle ; des radicaux cycloalkyle et cycloalkényle tels que par exemple des radicaux cyclohexyle, cycloheptyle, cyclohexényle ; des radicaux alkényle, par exemple des radicaux vinyle, allyle ;

des radicaux alkaryle ; des radicaux cyanoalkyle tels que par exemple un radical cyanoéthyle ; des radicaux halogénoalkyle, halogénoalkényle et halogénoaryle, tels que par exemple des radicaux chlorométhyle, trifluoro-3,3,3 propyle, chlorophényle, dibromophényle, trifluorométhylphényle ;
- le symbole R' représente un atome d'hydrogène, un radical alkyle ayant de 1 à 4 atomes de carbone, le radical bétaméthoxy-éthyle.

De préférence, le constituant (a) consiste dans un composé THV dont la viscosité à 25 °C est supérieure à $9.10^4$ mPa.s et plus précisément comprise entre $1.10^5$ mPa.s et $20.10^6$ mPa.s et dont au moins 60 % des symboles R représentent des radicaux méthyle. La présence, le long de la chaîne polydiorganosiloxane, de faibles quantités de motifs autres que $(R)_2SiO$, par exemple de motifs de formule $RSiO_{1,5}$ et/ou $SiO_2$ n'est cependant pas exclue dans la proportion d'au plus 2 % en mole par rapport au nombre de motifs $(R)_2SiO$.

A titre d'exemples concrets de motifs de formules $(R)_2SiO$ et $(R)_3SiO_{0,5}$ et de radicaux de formule OR', peuvent être cités ceux de formules :

$(CH_3)_2SiO$, $CH_3(CH_2=CH)SiO$, $CH_3(C_6H_5)SiO$, $(C_6H_5)_2SiO$,
$CH_3(C_2H_5)SiO$, $(CH_3CH_2CH_2)CH_3SiO$, $CH_3(n.C_3H_7)SiO$,
$(CH_3)_3SiO_{0,5}$, $(CH_3)_2CH_2=CHSiO_{0,5}$, $CH_3(C_6H_5)_2SiO_{0,5}$,
$(CH_3)(C_6H_5)(CH_2=CH)SiO_{0,5}$,
$-OH$, $-OCH_3$, $-OC_2H_5$, $-O-n.C_3H_7$, $-O-iso.C_3H_7$, $-O-n.C_4H_9$,
$-OCH_2CH_2OCH_3$.

Ces composés THV sont commercialisées par les fabricants de silicone ou peuvent être fabriquées en opérant selon des techniques déjà connues.

Les compositions cosmétiques selon la présente invention font appel encore, obligatoirement, à un constituant (b) consistant dans un composé silicone fluide ayant une viscosité à 25 °C au plus égale à 50 000 mPa.s. Ce composé silicone fluide peut être ($b_1$) un polydiméthysiloxane linéaire, ($b_2$) un polydiméthylsiloxane cyclique ou ($b_3$) un mélange de plusieurs espèces ($b_1$) ou ($b_2$) entre elles ou un mélange d'une ou plusieurs espèce(s) ($b_1$) avec une ou plusieurs espèce(s) ($b_2$). Le composé (b) est engagé dans des proportions, représentant le pourcentage pondéral de composé silicone fluide (b) dans la matière silicone (a) + (b), allant de 50 à 95 %.

Les polydiméthylsiloxanes linéaires ($b_1$) sont amplement décrits dans la littérature et ils sont disponibles dans le commerce. On préfère utiliser les polydiméthylsiloxanes linéaires qui sont bloqués à chacune des extrémités de leur chaîne par un motif triméthylsiloxyle. Toutefois des polymères bloqués autrement, par exemple par un groupe hydroxyle ou par un motif $(R''0)_3SiO_{0,5}$ avec R'' étant un radical alkyle ayant de 1 à 3 atomes de carbone, ne sont pas exclus. Par polymères ($b_1$) linéaires, on entend des polymères ne présentant, dans la chaîne, pas plus de 3 % en nombre de motifs siloxane autres que les motifs diméthylsiloxy. Il est entendu que par l'expression "polydiméthylsiloxane linéaire", on entend définir, pour le moins, l'hexaméthyldisiloxane.

Les polydiméthylsiloxanes cycliques ($b_2$) possèdent la formule générale suivante :

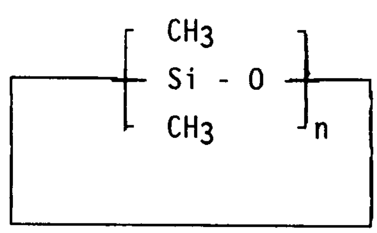

(I)

dans laquelle n est un nombre compris entre 3 et 15. On préfère utiliser les polydiméthylsiloxanes cycliques présentant pour n des valeurs allant de 4 à 7. Les espèces ($b_2$) et leur procédé de préparation sont décrits dans la littérature ; par ailleurs la plupart sont disponibles dans le commerce, c'est le cas en particulier pour les espèces $D_4$ (polydiméthylsiloxane cyclique avec n = 4), $D_5$ (polydiméthylsiloxane cyclique avec n = 5) et leurs divers mélanges possibles.

La viscosité du composé silicone fluide (b) peut varier dans de larges limites dès lors qu'elle est au plus égale à 50 000 mPa.s à 25 °C. Plus précisément, cette viscosité sera choisie dans l'intervalle précité, en tenant compte d'une part de la viscosité du composé THV (a) et d'autre part des proportions respectives de composé THV (a) et de composé (b), de manière à conduire à une matière silicone obtenue par

mélange de (a) + (b) qui soit suffisamment fluide pour être manipulable et aisément transformable en émulsion aqueuse fine et stable, c'est-à-dire une matière silicone (a) + (b) qui présente une viscosité inférieure à $5.10^5$ mPa.s, de préférence inférieure à $1.10^5$ mPa.s et, de façon encore plus préférée, comprise entre 80 et $5.10^4$ mPa.s à 25 C.

L'agent tensio-actif (c) utilisé conformément à la présente invention est un sucroglycéride.

Il est connu que par le terme "sucroglycérides", on désigne le mélange de produits directement obtenus par transestérification entre le saccharose et des triglycérides naturels ou de synthèse ; ce mélange contient des monoglycérides, des diglycérides, des triglycérides inaltérés (en faibles quantités), des monoesters et des diesters de saccharose.

Par triglycéride, on entend un ou plusieurs triglycérides d'acides gras aliphatiques saturés ou insaturés ayant au moins 12 atomes de carbone, de préférence 14 à 22 atomes de carbone. On peut évidemment partir d'un triglycéride de synthèse obtenu par réaction du glycérol et d'acides gras mais il est plus intéressant, pour des raisons économiques, de faire appel aux triglycérides naturels qui sont des mélanges.

Comme triglycérides naturels, on peut citer à titre d'exemples : le saindoux, le suif, l'huile d'arachide, l'huile de beurre, l'huile de graines de coton, l'huile de lin, l'huile de noix de coco, l'huile d'olive, l'huile de palme, l'huile de pépins de raisin, l'huile de poisson, l'huile de soja, l'huile de ricin, l'huile de coprah, l'huile de colza.

On emploie préférentiellement les sucroglycérides ($c_1$) d'huile de palme, de ricin, de coprah ou de colza.

Les sucroglycérides se présentent sous forme liquide (sucroglycérides de ricin et de colza) ou sous forme de pâtes plus ou moins consistantes se différenciant commercialement par leur point de fusion :

| | |
|---|---|
| - sucroglycérides de saindoux | 47 à 50 °C |
| - sucroglycérides de suif | 50 à 55 °C |
| - sucroglycérides d'huile de palme | 55 à 58 °C |
| - sucroglycérides d'huile de coprah | 30 à 32 °C |

Un mode préparatoire particulièrement adapté à ces sucroglycérides est décrit dans FR-A-2 463 152.

Pour préparer l'émulsion silicone/eau, forme sous laquelle se présentent les compositions cosmétiques selon l'invention, divers procédés sont utilisables.

Par exemple, quand on souhaite préparer une émulsion de type huile-dans-eau, deux modes d'exécution peuvent être réalisés qui consistent :

- soit (mode n° 1 de l'émulsion directe) à introduire la matière silicone obtenue par mélange de (a) + (b) dans l'eau en opérant sous agitation et à une température comprise entre la température ambiante et 60 °C, l'agent tensio-actif (c) ayant été préalablement dissous ou dispersé soit dans la matière silicone, soit dans l'eau ;
- sout (mode n° 2 de mise en émulsion par inversion de phases) à introduire l'eau dans la matière silicone obtenue par mélange de (a) + (b) en opérant sous agitation et à une température comprise entre la température ambiante et 60 °C, l'agent tensio-actif (c) ayant été préalablement dissous ou dispersé soit dans la matière silicone, soit dans l'eau ; dans ce second mode, on forme d'abord une émulsion eau-dans-huile qui s'inverse au cours de l'introduction d'eau lorsque sa quantité est suffisante.

Quand on souhaite préparer une émulsion de type eau-dans-huile, on peut opérer selon le mode n° 2 dont on vient de parler, mais en arrêtant cette fois l'introduction d'eau avant l'inversion de phase.

L'émulsion aqueuse ainsi obtenue est particulièrement fine et stable et se dilue ensuite aisément dans l'eau.

Les compositions cosmétiques conformes à l'invention, sont plus particulièrement destinées à être appliquées sur les cheveux ou la peau elles comprennent de préférence :

- 100 parties en poids de matière silicone (a) + (b) dans laquelle le pourcentage pondéral de composé THV (a) dans la matière silicone représente 10 à 30 %,
- 10 à 200 parties en poids d'agent tensio-actif (c), et
- 40 à 1 200 parties en poids d'eau, avec la condition supplémentaire selon laquelle le rapport parties en poids d'agent tensio-actif (c)/parties en poids d'eau est compris entre 0,05 et 0,4.

Ces compositions peuvent également renfermer au moins un solvant cosmétiquement acceptable et se présenter sous forme épaissie ou non, de crème, de lait, de gel, et être pressurisées en aérosol sous forme de mousse et de spray.

Elles peuvent plus particulièrement être utilisées comme shampooing, comme composition après-shampooing, comme produit à rincer applicable après shampooing, avant ou après coloration et décoloration, avant ou après permanente ou défrisage, comme lotion de mise en plis ou de brushing, comme composition restructurante ou additif aux permanentes ou comme composition de soins pour la peau.

Elles peuvent également être utilisées en dermatologie, auquel cas, elles contiennent une substance active sur le plan dermatologique.

Les compositions conformes à l'invention peuvent contenir encore au moins un adjuvant choisi parmi ceux habituellement utilisés en cosmétologie, tels que des corps gras comme par exemple des huiles minérales, des huiles d'origine animale et/ou des esters gras, des parfums, des colorants, des agents conservateurs, des agents hydratants, des agents séquestrants, des agents filtrants, des agents moussants, des agents de conditionnement, tels que par exemple des polymères et, notamment, des polymères cationiques (polymères comportant par exemple un grand nombre de groupements amonium quaternaire), anioniques (polymères dérivés par exemple d'acides carboxyliques insaturés éthyléniquement), non-ioniques ou amphotères ou leurs mélanges, des agents épaississants, des agents structurants, des stabilisateurs de mousse, des propulseurs ou autres adjuvants habituellement utilisés dans les compositions pour les cheveux ou la peau, suivant l'application envisagée.

Le pH des compositions selon l'invention est généralement compris entre 4 et 10 et, de préférence, entre 5 et 8,5. Il peut être ajusté à l'aide d'agents alcalinisants ou acidifiants bien connu dans la technique.

Les agents épaississants dont on a parlé ci-avant peuvent être choisis par exemple parmi les gommes de xanthane, la gomme de guar ou ses dérivés, la gomme arabique ou de caroube, l'alginate de sodium, les dérivés de cellulose, les dérivés d'acide polyacrylique. Ces agents épaississants, quand ils sont utilisés, sont présents dans des proportions comprises entre 0,1 et 30 % en poids par rapport au poids total de la composition.

Les agents propulseurs sont des propulseurs classiques tels que plus particulièrement les alcanes, les fluoroalcanes, les chlorofluoroalcanes ou leurs mélanges.

Les (ou les) solvant(s) et/ou adjuvant(s) cosmétiquement acceptable(s) peut (ou peuvent) être mis en oeuvre selon des techniques qui consistent :

- (i) soit à le (ou les) introduire dans les compositions de l'invention à un moment quelconque lors de la préparation de l'émulsion silicone/eau à partir des ingrédients (a), (b) et (c) et d'eau,
- soit (2i) à le (ou les) mélanger avec ladite émulsion une fois cette dernière obtenue, le mélange pouvant se faire soit par incorporation de l'émulsion dans le (ou les) solvant(s) et/ou adjuvant(s), soit par incorporation de ce (ou ces) dernier(s) dans l'émulsion.

A noter, dans le cadre de la technique (2i) précitée, que le (ou les) adjuvant(s) cosmétiquement acceptable(s) peut (ou peuvent) être engagé(s) sans inconvénient sous forme d'une émulsion aqueuse préparée de manière connue en soi en utilisant un agent tensio-actif conventionnel différent de l'ingrédient (c) selon la présente invention. A noter encore, dans le cadre de la technique (2i) mettant en oeuvre une émulsion adjuvant(s)/eau, qu'un autre adjuvant utilisable alors peut consister dans un composé silicone fluide choisi parmi les polydiméthylsiloxanes linéaires ($b_1$) dont on a parlé ci-avant dans le présent mémoire. La quantité d'eau apportée alors par l'émulsion adjuvant(s)/eau n'est pas critique dès lors que, additionnée à la quantité d'eau apportée par l'émulsion silicone/eau obtenue à partir des ingrédients (a), (b) et (c), elle conduit à une quantité totale d'eau dans l'ensemble émulsion silicone/eau + émulsion adjuvant(s)/eau qui obéit à la définition donnée ci-avant au point 2 de la page 2 du présent mémoire ou ne s'écarte pas de plus de 20 % de la valeur des bornes indiquées. De la même manière la quantité de matière silicone apportée éventuellement par l'émulsion adjuvant(s)/eau n'est pas critique dès lors que, additionnée à la quantité d'ingrédient (b) apportée par l'émulsion silicone/eau obtenue à partir des ingrédients (a), (b) et (c), elle conduit à une quantité totale d'ingrédient (b) dans l'ensemble émulsion silicone/eau + émulsion/adjuvant(s)/eau qui obéit à la définition donnée ci-avant au point 2 de la page 2 du présent mémoire on ne s'écarte pas de plus de 20 % de la valeur des bornes indiquées.

Le procédé de traitement cosmétique consiste essentiellement à appliquer une composition conforme à l'invention, soit sur les cheveux suivant l'usage envisagé (shampooing, traitement à rincer, traitement de coiffage sans rinçage), soit sur la peau (produits pour bain ou douche, produits bronzants, produits pour le rasage, lotions parfumées, crèmes ou laits).

Les exemples qui suivent sont destinés à illustrer l'invention sans pour autant présenter un caractère limitatif. Dans tout ce qui suit, les parties sont en poids, sauf mention contraire.

- **EXEMPLE 1** :

1. - Préparation d'une composition conforme à la présente invention :

On prépare une émulsion de type huile-dans-eau à partir des ingrédients suivants :

a) - 42 parties d'un composé THV polydiméthylsiloxane dont la chaîne linéaire est bloquée à chaque extrémité par un motif triméthylsiloxyle et qui possède une viscosité à 25 °C égale à $1.10^5$ ma.s ;

b) - 238 parties d'un mélange, 50/50 en poids, de polydiméthylsiloxanes cycliques D4 et $D_5$ possédant une viscosité à 25 °C égale à 3,6 mPa.s ;

c) - 120 parties de sucroglycéride d'huile de ricin ; cet ingrédient est le produit commercialisé par la Société RHONE-POULENC CHIMIE sous la marque déposée CELYNOL CO 11 ;

et - 600 parties d'eau.

La matière silicone (280 parties), obtenue par mélange de (a) + (b), possède une viscosité à 25 °C égale à 100 mPa.s.

Le sucroglycéride est dispersé dans 100 parties d'eau. Cette dispersion est versée ensuite dans la matière silicone (a) + (b) soumise à une agitation mécanique à l'aide d'un appareil dénommé ULTRA-TURRAX tournant à 13 500 tours/minute. On forme d'abord une émulsion de type eau-dans-huile qui s'inverse lorsque la quantité d'eau nécessaire à l'inversion de phase (100 parties) est ajoutée. L'émulsion de type huile-dans-eau ainsi obtenue est broyée à l'ULTRA-TURRAX (13 500 tours/minute) pendant 2 minutes. L'eau restante (400 parties) est alors incorporée lentement sous agitation modérée (agitateur de type pâle-cadre tournant à 500 tours/minute).

2. - Tests d'évaluation de l'émulsion :

2.1. - Stabilité en température : une fraction aliquote de l'émulsion qui vient d'être préparée est répartie dans 2 tubes de 50 $cm^3$ chacun que l'on ferme hermétiquement. Un tube est placé dans une étuve portée à 40 °C, tandis que l'autre tube est conservé à la température ambiante (23 °C). Par observation visuelle on note dans le temps l'absence des formes d'instabilité suivantes : le crèmage (déplacement des globules dispersés vers le haut), la sédimentation (déplacement des globules dispersés vers le bas) et la floculation (formation d'agrégats résultant de l'association d'un plus ou moins grand nombre de globules de la phase dispersée).

Résultats :

| TEMPERATURE DE STOCKAGE | 40 °C | 23 °C |
|---|---|---|
| Temps de stockage | 1 mois | > 1 mois |
| Comportement | stable | stable |

2.2. - Granulométrie des globules de la phase huileuse dispersée : la distribution granulométrique de l'émulsion est déterminée à l'aide du granulomètre à diffraction laser dénommé SYMPATEC. La mesure est faite 2 heures après l'émulsification.

Résultats : les limites supérieures des classes granulométriques, pour 10, 50 et 80 % de globules en volume, sont respectivement de : 1,23 µm (10 %), 3,23 µm (50 %) et 6 µm (80 %).

- **EXEMPLES 2 A 5** :

1. - Préparation de compositions conformes à la présente invention :

On opère selon le mode opératoire qui est décrit dans l'exemple 1, paragraphe 1 à partir des ingrédients dont la nature et les proportions (en parties) sont rassemblées dans le tableau donné ci-après :

| Ingrédient / Exemple | (a) : composé THV de l'ex. 1 | (b) : mélange D4 + D5 de l'ex. 1 | (c) : sucroglycéride de l'ex. 1 | EAU | | |
|---|---|---|---|---|---|---|
| | | | | (1) | (2) | (3) |
| 2 | 45 | 255 | 40 | 660 | 100 | 100 |
| 3 | 45 | 255 | 80 | 620 | 100 | 100 |
| 4 | 67,5 | 382,5 | 50 | 500 | 100 | 100 |
| 5 | 94,5 | 535,5 | 70 | 300 | 100 | 100 |

(1) : quantité totale d'eau ; (2) : quantité d'eau utilisée pour la dispersion du sucroglycéride ; (3) : quantité d'eau nécessaire à l'inversion de phase.

2. - Tests d'évaluations :

2.1. - Stabilité en température : les résultats s'expriment, pour chaque exemple 2 à 5, de la même façon que ceux obtenus dans l'exemple 1.

2.2. - Granulométrie des globules dispersés :

| EXEMPLE | % de globules en volume | | |
|---|---|---|---|
| | 10 | 50 | 80 |
| 2 | 0,93 µm | 2,40 µm | 4,20 µm |
| 3 | 0,94 µm | 2,54 µm | 5,00 µm |
| 4 | 1,05 µm | 2,47 µm | 4,20 µm |
| 5 | 1,24 µm | 2,81 µm | 4,50 µm |

- **EXEMPLE 6** :

1. - Préparation d'une composition conforme à la présente invention :

On prépare une émulsion de type huile-dans-eau à partir des ingrédients et adjuvant suivants :

a) - 45 parties d'un composé THV polyddiméthylsiloxane dont la chaîne linéaire est bloquée à chaque extrémité par un motif triméthylsiloxyle et qui possède une viscosité à 25 °C égale à $5.10^6$ mPa.s ;

b) - 255 parties d'un mélange, 50/50 en poids, de polydiméthylsiloxane cycliques $D_4$ et $D_5$ possédant une viscosité à 25 °C égale à 3,6 mPa.s ;

c) - 40 parties de sucroglycéride d'huile de ricin commercialisé par la Société RHONE-POULENC CHIMIE sous la marque déposée CELYNOL CO 11 ;

adjuvant - 2 parties de gomme xanthane ; il s'agit là du produit commercialisé par la Société RHONE-POULENC CHIMIE sous la marque déposée RHODOPOL SC ;

et - 658 parties d'eau.

La matière silicone (300 parties), obtenue par mélange de (a) + (b), possède une viscosité à 25 °C égale à 3 530 mPa.s.

Le sucroglycéride est dispersé dans 100 parties prélevées dans la phase aqueuse formée par le mélange de la gomme xanthane (2 parties) et d'eau (658 parties). Cette dispersion est versée ensuite dans la matière silicone (a) + (b) soumise à une agitation mécanique comme indiqué ci-avant dans l'exemple 1, paragraphe 1. L'émulsion de type eau-dans-huile obtenue est inversée par ajout, toujours sous agitation, de la quantité nécessaire de phase aqueuse (100 autres parties). L'émulsion de type huile-dans-eau qui est formée ainsi que le reste de la phase aqueuse (460 parties) sont introduits dans un moule colloïdal dénommé FRYMA et l'ensemble est broyé pendant 5 minutes sous pression réduite.

2. - Test d'évaluation de l'émulsion :

2.1. - Stabilité en température : une fraction aliquote de l'émulsion est répartie dans 3 tubes de 50 $cm^3$ chacun. Un premier tube est stocké à température ambiante (23 °C) ; un second tube est stocké dans une étuve portée à 40 °C ; le troisième tube est stocké dans une enceinte dans laquelle la température est soumise à des cycles de températures (24 heures à 5 °C ; 24 heures à 40 °C). Après 1 mois de stockage, et pour chacune des conditions de stockage précitées, l'observation visuelle ne permet pas de déceler des formes d'instabilité telles que le crèmage, la sédimentation, la floculation.

2.2. - Granulométrie : la mesure est faite, selon le protocole décrit dans l'exemple 1, paragraphe 2.2., sur l'émulsion stockée pendant 1 mois à température ambiante.

Résultats : Les limites supérieures des classes granulométriques, pour 10, 50 et 80 % de globules en volume, sont respectivement de : 0,93 µm (10 %), 2,08 µm (50 %) et 4,20 µm (80 %).

- **EXEMPLE 7** :

1. - Préparation d'une composition conforme à la présente invention :

On prépare une émulsion de type huile-dans-eau à partir des ingrédients et adjuvant suivants :

a) - 45 parties d'un composé THV polydiméthylsiloxane dont la chaîne linéaire est bloquée à chaque extrémité par un motif triméthylsiloxyle et qui possède une viscosité à 25 °C égale à $5.10^6$ mPa.s ;

b) - 255 parties de polydiméthylsiloxane cyclique $D_5$ possédant une viscosité à 25 °C égale à 4,7 mPa.s ;

c) - 40 parties de sucroglycéride d'huile de colza commercialisée par la Société RHONE-POULENC CHIMIE sous la marque déposée CELYNOL SGF ;

adjuvant - 2 parties de gomme xanthane commercialisée par la Société RHONE-POULENC CHIMIE sous la marque déposée RHODOPOL SC ;

et - 658 parties d'eau.

L'émulsion est préparée directement en dispersant, à température ambiante (23 °C), la matière silicone (300 parties), obtenue par mélange de (a) + (b), dans 660 parties de phase aqueuse formée par le mélange de la gomme xanthane (2 parties) et d'eau (658 parties).

Le sucroglycéride (c) a été préalablement dispersé dans la matière silicone (a) + (b), laquelle possède une viscosité à 25 °C égale à 3 640 mPa.s.

La dispersion de la matière silicone dans la phase aqueuse est conduite sous agitatior mécanique en opérant au sein du moulin colloïdal FRYMA. Après ajout de la matière silicone, le contenu du moulin est broyé pendant 3 minutes sous pression réduite.

2. - Test d'évaluation de l'émulsion :

2.1. - Stabilité en température : après 8 mois de stockage à température ambiante (23 °C), l'observation visuelle ne permet pas de déceler des formes d'instabilité telles que le crèmage, la sédimentation, la floculation.

2.2. - Granulométrie : la mesure est faite, selon le protocole décrit dans l'exemple 1, paragraphe 2.2., sur l'émulsion stockée pendant 8 mois à température ambiante.

Les limites supérieures des classes granulométriques, pour 10, 50 et 80 % des globules en volume, sont respectivement de : 1,29 µm (10 %), 2,74 µm (50 %) et 4,20 µm (80 %).

- **EXEMPLE 8** :

1. - Préparation d'une composition conforme à la présente invention, se présentant sous forme de creme, à partir des ingrédients (a), (b) et (c), d'eau et d'adjuvants en utilisant la technique (2i) décrite ci-avant à la page 10 du présent mémoire :

1.1. - Préparation de l'émulsion silicone/eau : on prépare une émulsion de type huile-dans-eau à partir des ingrédients et adjuvants suivants :

a) - 45 parties d'un composé THV polydiméthylsiloxane dont la chaîne linéaire est bloquée à chaque extrémité par un motif triméthylsiloxyle et qui possède une viscosité à 25 °C égale à $5.10^6$ mPa.s ;

b) - 255 parties d'un mélange, 50/50 en poids, de polydiméthylsiloxanes cycliques $D_4$ et $D_5$ possédant une viscosité à 25 °C égale à 3,6 mPa.s ;

c) - 40 parties de sucroglycéride d'huile de ricin commercialisée par la Société RHONE-POULENC CHIMIE sous la marque déposée CELYNOL CO 11 ;

adjuvants - . 2 parties de gomme xanthane commercialisée par la Société RHONE-POULENC CHIMIE sous la marque déposée RHODOPOL SC, et . 10 parties de propylèneglycol (ou dihydroxy-1,2 propane) ;

et - 648 parties d'eau.

Pour préparer l'émulsion souhaitée, on procède d'abord à la dispersion du sucroglycéride dans la matière silicone (300 parties) obtenue par mélange de (a) + (b) ; ladite matière silicone possède une viscosité à 25 °C égale à 3 530 mPa.s. On introduit ensuite dans la dispersion sucroglycéride/matière silicone 100 parties de la phase aqueuse formée par le mélange de la gomme xanthane (2 parties), de propylèneglycol (10 parties) et d'eau (648 parties) ; cette introduction est conduite sous agitation mécanique (ULTRA-TURRAX tournant à 13 500 tours/minute). L'émulsion de type eau-dans-huile obtenue est inversée par ajout, toujours sous agitation, de la quantité nécessaire de phase aqueuse (100 autres parties). L'émulsion de type huile-dans-eau qui est formée est broyée à l'ULTRA-TURRAX (13 500 tours/minute) pendant 5 minutes. La phase aqueuse résiduelle (460 parties) est ajoutée sous agitation modérée (agitateur pâle-cadre tournant à 500 tours/minute).

1.2. - Préparation de la crème : la crème est obtenue par introduction, en opérant sous agitation mécanique (agitateur pâle-cadre tournant à 500 tours/minute) :

- de 15 parties de l'émulsion silicone/eau qui vient d'être décrite au paragraphe 1.1.,
- dans 85 parties d'une émulsion adjuvants/eau épaisse, à phase continue aqueuse, maintenue à 50 °C.

L'émulsion adjuvants/eau est préparée par mélange d'eau (71 parties) et des adjuvants suivants : 10 parties d'huile de vaseline, 3 parties d'acide stéarique et 16 parties d'un mélange de stéarates de polyoxyéthylèneglycols commercialisé par la Société GATTEFOSSE sous la marque déposée TEFOSE.

2. - Granulométrie des globules de la phase huileuse dispersée de la crème :

La mesure est faite selon le protocole décrit dans l'exemple 1, paragraphe 2.2.

On trouve dans la crème que 50 % en volume des globules dispersés ont des dimensions qui sont inférieures à 3,5 µm.

## Revendications

**1.** Compositions cosmétiques se présentant sous forme d'émulsions aqueuses silicone/eau, caractérisées par les points suivants :

- point 1 : elles sont préparées en milieu aqueux à partir de constituants comprenant les ingrédients obligatoires suivants :

  (a) : un composé THV (THV = très haute viscosité) polydiorganosiloxane dont la viscosité à 25 °C est au moins égale à $9.10^4$ mPa.s,

  (b) : au moins un composé silicone fluide dont la viscosité à 25 °C est au plus égale à 50 000 mPa.s,

  (c) : un agent tensio-actif non ionique consistant en un sucroglycéride ;
- point 2 : elles comportent :

- 100 parties en poids de matière silicone constituée par le mélange (a) + (b), le pourcentage pondéral de composé THV (a) dans la matière silicone (a) + (b) représentant 5 à 50 %,
- 2 à 700 parties en poids d'agent tensio-actif (c), et
- 3 à 2 000 parties en poids d'eau, avec la condition supplémentaire selon laquelle le rapport parties en poids d'agent tensio-actif (c)/parties en poids d'eau est au plus égal à 0,7.

**2.** Compositions selon la revendication 1, caractérisées en ce que le constituant (a) consiste dans un composé THV polydiorganosiloxane dont la chaîne est constituée essentiellement de motifs de formule $(R)_2SiO$ et est bloquée à chaque extrémité par un motif de formule $(R)_3SiO_{0.5}$ et/ou un radical de formule OR', formules dans lesquelles :
- les symboles R, identiques ou différents, représentent des radicaux hydrocarbonés monovalents, alkyle, des radicaux aryle, des radicaux aralkyle, des radicaux cycloalkyle et cycloalkényle, des radicaux alkényle, des radicaux alkaryle, des radicaux cyanoalkyle, des radicaux halogénoalkyle, halogénoalkényle et halogénoaryle,
- le symbole R' représente un atome d'hydrogène, un radical alkyle ayant de 1 à 4 atomes de carbone, le radical bétaméthoxy-éthyle.

**3.** Compositions selon la revendication 2, caractérisées en ce que le constituant (a) consiste dans un composé THV dont la viscosité à 25 °C est comprise entre $1.10^5$ et $20.10^6$ mPa.s et dont au moins 60 % des symboles R représentent des radicaux méthyle.

**4.** Compositions selon l'une quelconque des revendications 1 à 3, caractérisées en ce que le constituant (b) consiste dans ($b_1$) un polydiméthysiloxane linéaire, ($b_2$) un polydiméthylsiloxane cyclique ou ($b_3$) un mélange de plusieurs espèces ($b_1$) ou ($b_2$) entre elles ou un mélange d'une ou plusieurs espèce(s) ($b_1$) avec une ou plusieurs espèce(s) ($b_2$).

**5.** Compositions selon la revendication 4, caractérisées en ce que :
- les polydiméthylsiloxanes linéaires ($b_1$) sont bloqués à chacune des extrémités de chaîne par un motif triméthysiloxyle ;
- les polydiméthylsiloxanes cycliques ($b_2$) comportent de 4 à 7 atomes de silicium.

**6.** Compositions selon l'une quelconque des revendications 1 à 5, caractérisées en ce que la viscosité du constituant (b), qui est au plus égale à 50 000 mPa.s à 25 °C, est choisie dans l'intervalle précité, en tenant compte d'une part de la viscosité du composé THV (a) et d'autre part des proportions respectives du composé THV (a) et de constituant (b), de manière à conduire à une matière silicone obtenue par mélange de (a) + (b) dont la viscosité à 25 °C est inférieure à $1.10^5$ mPa.s.

**7.** Compositions selon l'une quelconque des revendications 1 à 6, caractérisées en ce que l'on utilise, comme agent tensio-actif (c) appartenant à la famille des sucroglycérides, un sucroglycéride d'huile de palme, de ricin, de coprah ou de colza.

**8.** Compositions selon l'une quelconque des revendications 1 à 7, caractérisées en ce qu'elles renferment en outre au moins un solvant cosmétiquement acceptable.

**9.** Compositions selon l'une quelconque des revendications 1 à 8, caractérisées en ce qu'elles renferment en outre au moins un adjuvant cosmétiquement acceptable choisi parmi les corps gras, les parfums, les colorants, les agents conservateurs, les agents hydratants, les agents séquestrants, les agents filtrants solaires, les agents moussants, les agents de conditionnement, les agents épaississants, les agents structurants, les stabilisateurs de mousse, les propulseurs, et les autres adjuvants habituellement utilisés dans les compositions pour les cheveux ou la peau.

## Claims

**1.** Cosmetic compositions in the form of silicone/water agueous emulsions, characterized by the following points:
- point 1: they are prepared in an agueous medium from constituents comprising the following obligatory ingredients:

(a): a VHV (VHV = very high viscosity) polydiorganosiloxane compound whose viscosity at 25 °C is at least $9x10^4$ mPa s,
(b): at least one fluid silicone compound whose viscosity at 25 °C is not more than 50 000 mPa s,
(c): a nonionic surface-active agent consisting of a sugar glyceride;
- point 2: they comprise:
  - 100 parts by weight of silicone matter consisting of the mixture (a) + (b), the percentage by weight of VHV compound (a) in the silicone matter (a) + (b) representing 5 to 50 %,
  - 2 to 700 parts by weight of surface-active agent (c), and
  - 3 to 2000 parts by weight of water, with the additional condition according to which the ratio parts by weight of surface-active agent (c)/parts by weight of water is at most 0.7.

**2.** Compositions according to Claim 1, characterized in that the constituent (a) consists of a VHV polydiorganosiloxane compound whose chain consists essentially of units of formula $(R)_2SiO$ and is blocked at each end by a unit of formula $(R)_3SiO_{0.5}$ and/or a radical of formula OR', in which formulae:
- the symbols R, which are identical or different, denote monovalent hydrocarbon radicals, alkyl, aryl radicals, aralkyl radicals, cycloalkyl and cycloalkenyl radicals, alkenyl radicals, alkaryl radicals, cyanoalkyl radicals and haloalkyl, haloalkenyl and haloaryl radicals,
- the symbol R' denotes a hydrogen atom, an alkyl radical containing from 1 to 4 carbon atoms or the beta-methoxyethyl radical.

**3.** Compositions according to Claim 2, characterized in that the constituent (a) consists of a VHV compound whose viscosity at 25 °C is between $1x10^5$ and $20x10^6$ mPa s and in which at least 60 % of the symbols R denote methyl radicals.

**4.** Compositions according to any one of Claims 1 to 3, characterized in that the constituent (b) consists of ($b_1$) a linear polydimethylsiloxane, ($b_2$) a cyclic polydimethylsiloxane or ($b_3$) a mixture of a number of species ($b_1$) or ($b_2$) with each other or a mixture of one or more species ($b_1$) with one or more species ($b_2$).

**5.** Compositions according to Claim 4, characterized in that:
- the linear polydimethylsiloxanes ($b_1$) are blocked by a trimethylsiloxy unit at each of the ends of the chain;
- the cyclic polydimethylsiloxanes ($b_2$) contain from 4 to 7 silicon atoms.

**6.** Compositions according to any one of Claims 1 to 5, characterized in that the viscosity of the constituent (b), which does not exceed 50 000 mPa s at 25 °C, is chosen within the abovementioned range, taking into account, on the one hand, the viscosity of the VHV compound (a) and, on the other hand, the respective proportions of the VHV compound (a) and of constituent (b), so as to result in a silicone matter obtained by mixing (a) + (b) whose viscosity at 25 °C is lower than $1x10^5$ mPa s.

**7.** Compositions according to any one of Claims 1 to 6, characterized in that the surface-active agent, belonging to the class of sugar glycerides, which is employed is a sugar palm, castor, copra or rapeseed oil glyceride.

**8.** Compositions according to any one of Claims 1 to 7, characterized in that they additionally contain at least one cosmetically acceptable solvent.

**9.** Compositions according to any one of Claims 1 to 8, characterized in that they additionally contain at least one cosmetically acceptable adjuvant chosen from fatty substances, perfumes, colorants, preserving agents, hydrating agents, sequestering agents, sunscreen agents, foaming agents, conditioning agents, thickening agents, structuring agents, foam stabilisers, propellants and the other adjuvants usually employed in compositions for the hair or the skin.

**Patentansprüche**

**1.** Kosmetische Zusammensetzungen in Form von wäßrigen Emulsionen aus Silicon und Wasser, dadurch gekennzeichnet, daß die Zusammensetzungen:

1. in wäßrigem Medium aus folgenden notwendigen Inhaltsstoffen als Bestandteile hergestellt werden:

(a) einer Polydiorganosiloxan-Verbindung mit shV (shV = sehr hohe Viskosität), deren Viskosität bei 25 °C wenigstens gleich 9 • $10^4$ mPa • s ist,

(b) wenigstens einer dünnflüssigen Siliconverbindung, deren Viskosität bei 25°C höchstens gleich 50.000 mPa • s ist,

(c) einem nichtionischen oberflächenaktiven Mittel aus einem Saccharoseglycerid (Sucroglycerid);

2. ferner folgendes umfassen:

- 100 Gewichtsteile einer Siliconsubstanz, die aus der Mischung von (a) + (b) besteht, wobei der Gewichtsanteil der Verbindung mit shV (a) in der Siliconsubstanz (a) + (b) 5 bis 50 Gew.-% beträgt,
- 2 bis 700 Gewichtsteile des oberflächenaktiven Mittels (c), und
- 3 bis 2.000 Gewichtsteile Wasser, mit der zusätzlichen Bedingung, daß das Verhältnis von Gewichtsteilen des oberflächenaktiven Mittels (c) zu Gewichtsteilen Wasser höchstens gleich 0,7 ist.

**2.** Zusammensetzungen nach Anspruch 1 dadurch gekennzeichnet, daß der Bestandteil (a) aus einer Polydiorganosiloxan-Verbindung mit shV besteht, deren Kette im wesentlichen aus Einheiten der Formel $(R)_2SiO$ besteht und an jedem Ende durch eine Einheit der Formel $(R)_3SiO_{0.5}$ und/oder einem Rest der Formel OR' blockiert wird, wobei in den Formeln:

- die Symbole R gleich oder unterschiedlich sind und einwertige Kohlenwasserstoff-, Alkyl-, Aryl-, Aralkyl-, Cycloalkyl- und Cycloalkenyl-, Alkenyl-, Alkaryl-, Cyanoalkyl-, Halogenalkyl-, Halogenalkenyl- und Halogenarylgruppen darstellen können,
- das Symbol R' ein Wasserstoffatom, eine Alkylgruppe mit 1 bis 4 Kohlenstoffatomen, oder eine $\beta$-Methoxyethylgruppe darstellt.

**3.** Zusammensetzungen nach Anspruch 2, dadurch gekennzeichnet, daß der Bestandteil (a) aus einer Verbindung mit shV besteht, deren Viskosität bei 25°C zwischen 1 • $10^5$ und 20 • $10^6$ mPa • s liegt und wenigstens 60% der R-Reste Methylgruppen darstellen.

**4.** Zusammensetzungen nach einem der Ansprüche 1 bis 3, dadurch gekennzeichnet, daß der Bestandteil (b) aus ($b_1$) einem linearen Polydimethylsiloxan, ($b_2$) einem cyclischen Polydimethylsiloxan oder ($b_3$) einer Mischung aus mehreren Typen ($b_1$) oder ($b_2$) untereinander oder einer Mischung eines Typs oder mehrerer Typen ($b_1$) mit einem oder mehreren Typ(en) ($b_2$) besteht.

**5.** Zusammensetzungen nach Anspruch 4, dadurch gekennzeichnet, daß:

- die linearen Polydimethylsiloxane ($b_1$) an jedem Ende der Kette durch eine Trimethylsiloxyleinheit blockiert werden; und
- die cyclischen Polydimethylsiloxane ($b_2$) 4 bis 7 Siliciumatome enthalten.

**6.** Zusammensetzungen nach einem der Ansprüche 1 bis 5, dadurch gekennzeichnet, daß die Viskosität des Betandteils (b), die höchstens gleich 50.000 mPa • s bei 25 °C ist, aus dem vorstehenden Bereich ausgewählt wird, wobei einerseits die Viskosität der Verbindung mit shV (a) und andererseits die jeweiligen Anteile der Verbindung mit shV (a) und des Bestandteils (b) berücksichtigt werden, so daß eine Siliconsubstanz durch Mischen von (a) + (b) erhalten wird, deren Viskosität bei 25 °C kleiner als 1 • $10^5$ mPa • s ist.

**7.** Zusammensetzungen nach einem der Ansprüche 1 bis 6, dadurch gekennzeichnet, daß als oberflächenaktives Mittel (c) aus der Familie der Sucroglyceride ein entsprechendes Derivat von Palmöl, Rizinusöl, Copraöl oder Rapsöl verwendet wird.

**8.** Zusammensetzungen nach einem der Ansprüche 1 bis 7, dadurch gekennzeichnet, daß sie ferner wenigstens ein für Kosmetika zulässiges Lösemittel beinhalten.

**9.** Zusammensetzungen nach einem der Ansprüche 1 bis 8, dadurch gekennzeichnet, daß sie ferner wenigstens ein für Kosmetika zulässiges Hilfsmittel beinhalten, das ausgewählt wird aus Fetten, Parfums, Färbemitteln, Konservierungsmitteln, Hydratationsmitteln, Komplexbildnern, Sonnenfiltern, Schaumbildnern, Konditionierungsmitteln, Verdickungsmitteln, Strukturierungsmitteln, Schaumstabilisatoren, Treibmitteln und anderen Additiven, die gewöhnlich in Zusammensetzungen fuhr die Haare oder die Haut verwendet werden.